# EUROPEAN PATENT SPECIFICATION

(11) **EP 1 813 352 B1**
(45) Date of publication and mention of the grant of the patent: **15.05.2013**
(21) Application number: 05799033.5
(22) Date of filing: 21.09.2005
(51) Int. Cl.: B05B 1/06

(54) **DEVICE FOR THE PNEUMATIC ATOMISATION OF LIQUIDS USING AN IMPLOSIVE GAS STREAM**
VORRICHTUNG ZUR DRUCKLUFTZERSTÄUBUNG VON FLÜSSIGKEITEN UNTER VERWENDUNG EINES IMPLOSIVEN GASSTROMS
DISPOSITIF D'ATOMISATION PNEUMATIQUE DE LIQUIDES AU MOYEN D'UN FLUX D'IMPLOSION GAZEUX

(30) Priority: 30.09.2004 ES 200402333
(43) Date of publication of application: 01.08.2007
(73) Proprietor: Universidad de Sevilla, E-41012 Sevilla (ES)
(72) Inventor: GAÑÁN CALVO, Alfonso, Miguel, Camino de los Descubrimientos s/n, E-41092 SEVILLA (ES); MENDOZA SIMÓN, Eladio Dpto. Ingenieria Energetica y Mecanica de Fluidos, Camino de los Descubrimientos s/n, E41092 SEVILLA (ES); RIESCO CHUECA, Pascual Dpto. Ingenieria Energetica, y Mecanica de Fluidos, Camino de los Descubrimientos s/n E-41092 SEVILLA (ES)
(74) Representative: Elzaburu Marquez, Alberto
(86) International application number: PCT/ES2005/000512
(87) International publication number: WO 2006/037823

(56) References cited:
- WO-A-00/76673
- WO-A-2005/070556
- DE-A1- 4 435 415
- FR-A- 993 145
- US-A- 4 162 282
- US-A- 5 046 667
- US-B1- 6 241 159
- US-B1- 6 409 098

## Description

### The object of the invention

The object of the current invention is a device and a procedure for the atomization or nebulization of a liquid by using an impulsion gas or steam (hereinafter, gas) pressurized into such device. Both fluids are expelled to the outside after their mixture, causing the liquid to exit as a spray or suspension of droplets that is carried by the gas flow. The device consists of a liquid chamber, contained in a vessel or pressurized bottle, and a liquid-gas mixing zone, where the combination between both phases and their exit occurs. The impulsion gas gets into the bottle through an injection inlet, subsequently leaving such vessel through the mixing zone. The free surface of the liquid within the bottle is pressurized by the impulsion gas, causing the liquid to be impelled to the mixing zone through a feeding tube whose nozzle is close to the bottom of the bottle. At the other end of the feeding tube, called nebulizer end, there is an exit hole. Said exit section is approximately opposed to a vessel exit orifice, through which the mixed liquid/gas goes out to the outside as a suspension of droplets. Said exit orifice is perforated at the wall of the vessel. A key feature of the invention is that the internal edges of such exit hole and the external edges of such exit orifice define two closed lines in approximately parallel planes and separated by a short distance; the passage surface comprised between both edge lines is ring-shaped. The gas coming from the pressurized bottle that tries to escape to the outer environment, flows in a essentially radial and centripetal pattern in the surroundings of the mixing zone, leading to the cross flow with the liquid flow coming from the feeding tube. Immediately after passing through that ring-shaped surface, the gas intercepts said liquid flow at the perimeter and in an essentially perpendicular direction. The minimum passage section of the approximately radial flow of the exiting gas is indeed located at the ring-shaped surface; said minimum section has a surface of the same order as the section of the exit orifice. The feeding tube may have a pressure drop adjuster, which allows controlling the flow rate of the atomized liquid.

### Introduction to the state of the art

A device according to the preamble of claim 1 is known from US 5046667.

Nebulizers enable the transformation of a liquid in a spray or suspension of microdroplets. Nebulizers usually consist of a reserve tank where the liquid is introduced, a nebulization chamber where the spray is generated, and an energy supply, generally a pump, to impel the carrier gas of the suspension. The atomization of liquids by purely fluid dynamics means, and particularly through pneumatic means, is an essential operation in multiple industrial, technological, scientific applications and developments and in the daily life. Sprays have been used in numerous technological fields, particularly as a way to treat diseases of respiratory tracks through the nebulization of liquid medicaments. Spray drug delivery by means of inhalation leads to the adequate medicament concentration in the respiratory track, minimizing secondary effects.

Applications in the agricultural field are also very extended for pesticide atomization, for instance in insecticide treatments. For that purpose, manual and automatic equipments are used (portable, vehicle mounted), allowing aim-targeted delivery and capability to control the drop size, whose diameter is usually between 100 and 500 microns. When drop sizes are smaller, between 50 and 100 microns, the term nebulization is commonly used: for insecticide applications, it increases the flotation capability of the preparation and also the liquid contact area when drop deposition takes place.

The atomization of liquids is based on diverse technological principles. These principles mark the quality and stability of the spray (monodispersity, drop size), as well as the use friendliness and procedure economy.
- Centrifugal atomization: it is the most extended, it uses a wheel or rotating disk to break the liquid flow into droplets. Depending on the spin velocity, different droplet sizes are achieved. There are centrifugal atomizers of every size in the market, from lab scale to the great industrial scale.
- Pressurized hydrodynamic atomization: the pressure drop on a nozzle or the gravitatory effects cause liquid breakup. Generally, the liquid is impelled through a narrow nozzle. As a result, operation is poor when the liquid causes abrasion of the nozzle or when there is sedimentation altering the ejection geometry. Most of industrial applications are based on this principle: in-door humidification, micro irrigation, surface treatments of steel and sheets, paint application.
- Pneumatic atomization: A second fluid, generally a gas, is used to facilitate the liquid atomization. Shear stress between the gas and the liquid cause the disintegration of the liquid in droplets. In general, pneumatic atomization achieves good performance with moderate pressures. Some examples are pharmaceutical inhalers. Inhalers usually consist of a chamber with the medicament in liquid state, an air stream, a feeding tube for the medicament, and an impact plate combined with a deflector. Compressed air is injected, causing a Venturi effect when circulating at a high velocity through a narrowing. The resulting pressure drop sucks liquid from the liquid chamber. When the medical preparation and the air flow reach the impact plate, the liquid is broken into droplets of different sizes. The largest among them impact against the deflector and are returned to the liquid chamber, while the smaller ones are carried by the air flow and exit to the outside. The impelling gas can be CFCs (Chlorofluorocarbon), with the subsequent environmental uncertainty.

- Electrohydrodynamic atomization of liquids (*electrospray*): it is an essential tool of the biochemical analysis (Electrospray Mass Spectrometry, or ESMS), ever since it was discovered about the 80s. One of its advantages is the minimum quantity of analite that is required for the analysis. However, for applications where a sufficiently large liquid flow rate is to be atomized, one of the problems of electrospray is its low productivity. Examples of this type of applications can be found in the pharmaceutical field (drug encapsulation), agroalimentary (encapsulation of different organoleptic ingredients) and phytosanitary industry.
- Ultrasonic atomization: not yet very extended, it is based in the circulation of a liquid over a surface that vibrates at a high frequency. It can generate very small droplets with low flow rates.

The so-called Flow Focusing (FF) technology (Gañán-Calvo 1998, Physical Review Letters 80, 285), uses an especial geometry and the pneumatic means to generate liquid microjets; after exiting through an orifice these microjets break up into droplets with a small and substantially homogeneous size. This technology is also able to generate liquid microjets using a liquid instead of a gas, or to generate a gas microjet within a liquid (the same liquid or other different used as the focusing, i.e., with the same role played by the gas in the pneumatic procedure), leading to the generation of perfectly homogeneous microbubbles.

Subsequently, the patent WO 0076673 (D1) proposed a configuration of flow, then called "violent flow focusing"; unlike FF, the focusing gas has here an essentially radial and centripetal flow (*diaphragm-flow*), concentrically directed as a thin layer that intercepts the exit of the liquid at a flow surface transversal to the axis of movement of the liquid. As explained in D1, the gas comes from a pressure chamber, and the intense interaction produced between the liquid phase, whose movement is essentially axial, and the gas phase, directed radially, give rise to an immediate transfer of momentum. However, in D1, the liquid issues to the outer atmosphere as a jet.

As it is shown in the description below, the differentiating aspects of the present invention, whose principle will be referred to as *anti-flow-focusing* (AFF), in contrast with D1 (*violent-flow-focusing* o VFF), are the following:
- Free cross flow geometry, where it is not required that the gas diaphragm-flow passes through a narrow path of flat and convergent walls (see Figs. 1-5 of D1, in particular Fig. 2). The only requirement is that the exit hole and the exit orifice have two line edges separated by a short distance, defining a ring-shaped passage surface where the gas is forced to cross with an essentially radial and centripetal flow pattern. Thus there are no restrictions in the geometry previous to that minimum section.
- Special geometry which specifies additionally that the area relationship between the ring-shaped passage section of the radial gas flow (*diaphragm-flow*) and the passage section of the axial liquid flow (*piston flow*) is of the order of unity.

- Joint pressurization of both gas and liquid: a single pressure chamber at a single pressure (referred to as *impulsion pressure*) higher than the external environment pressure, where both phases are located before their mixture in the nebulization area.
- Selection of an adequate impulsion pressure so that the atomization of the liquid jet is generated in the interval comprised between the exit of the feeding tube transporting the liquid (called *exit hole*) and the exit to the outside of the two mixed phases through the so called *exit orifice.*

With regard to D1, of which the present patent is a later development, the hereby described invention introduces specifications to the design allowing the complete atomization of the liquid jet before its exit. It ensures at the same time a significant simplification of the design requiring only one pressurization element.

The present invention, belonging to the pneumatic atomizer field, intends to combine the advantages of a robust and simple design, with performance in continuous regime at low pressures by means of an impulsion gas that, in most cases, can be atmospheric air. The present invention allows using a gas-to-liquid mass flow ratio as low as one part of gas per seven parts of liquid, keeping an adequate atomization level of the liquid. Hence the device object of the present invention is very efficient from the energy point of view. The low energetic consumption of the device here described is compatible with a renewable energy source such as photovoltaic cell or wind power.

On the other hand, the patent *"Nebulizador neumático de válvula integrada"* P200401504 (D2) shows an atomizing device with the same general configuration as here described, based on the same combined liquid and gas pressurization principle, with the following differences:
- The local geometry of the flow in the meeting point between the liquid phase and the gas phase is not specified. Thus the possibility of choosing whichever flow mode remains open: flow-focusing, violent-flow-focusing (D1) or even procedures based on electrification (such as electrospray or a combination of electrospray and flow focusing, refer to the patent "*Dispositivo para la producción de chorros capilares y partículas micro- y nano-métricos*" PCT ES03/00065).
- The gas exits the pressurization bottle through an outlet tube independent of the feeding tube through which the liquid exits. This outlet tube is superfluous in the present invention.
- he device works in three flow regimes dependant on the position of the three way valve. Also this valve is superfluous with the present invention.

### Description of the invention

The invention relates to a device according to claim 1 and a procedure according to claim 17.

Below is described the method applied in the present patent, referred to as "Anti Flow Focusing" (hereafter AFF), which is suitable for the production of extremely thin aerosols and particles suspensions. This method originates from a later configuration of the flow that D1 describes as *violent flow-focusing.* In general, AFF is based in a geometric configuration that maximises the interaction between a liquid (dispersed phase) and a highly accelerated fluid (bearer phase). The AFF method optimizes the energy transference between both phases and, compared to other current pneumatic methods, reaches a significant increase of the proportion of energy used to generate surface in the dispersed phase.

Although any fluid, provided that it is sufficiently different from the dispersed phase (i.e. non mixable), can be used as bearer phase, usually this bearer phase will be air or any inert gas. Therefore, to simplify the description of the invention, from now on it will be referred to simply as "gas", not intending by it any restrictions to the range of fluids that can be used as a bearer phase.

The liquid is led to the area where the interaction with the gas takes place by means of a hermetic transport means preventing premature mixing of the two phases. The shape of said liquid transport means may vary substantially with no impact on the operation of the AFF; only the exit shape may be marginally influent because this is the area where the interaction between the two phases takes place. In order to simplify the invention description, hereafter we will refer to this transport means as "tube", thus not implying restrictions to the shape, number or configuration of the parts composing said transport means.

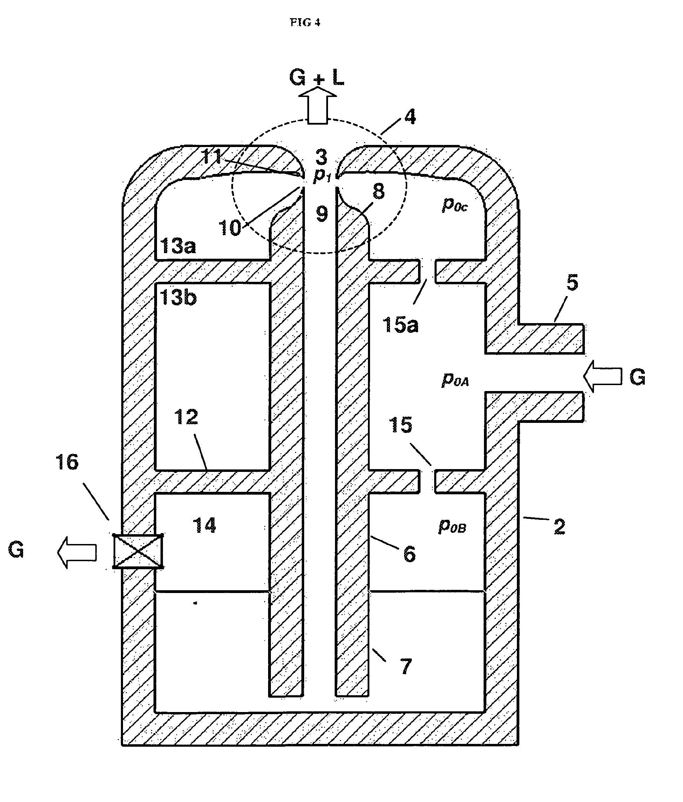

In AFF (see figure 4), a liquid flowing through a feeding tube (6) with a given flow rate, accesses through the exit hole (9) of said tube to a pressure chamber that is continuously filled in with gas. This pressure chamber has an exit orifice (3) through which the mixture of the bearer and dispersed phases exits. This orifice must be placed opposite to the exit hole (9) of the liquid tube and close to it, with a short axial difference between the facing edges of the orifice and hole, defining a section passage for the gas. The interaction between both phases takes place in the mixing region located between the mentioned hole and orifice. The gas that, owing to its pressure, tries to exit to the exterior, must cross previously that passage section defined by the ring-shape interval comprised between the facing borders of the exit orifice and exit hole; this fact drives the gas with an orientation essentially radial and perpendicular to the liquid movement axis when it exits the tube; this gas movement intercepting the liquid flow centripetally is named *diaphragm-flow*; the minimal passage section for the gas on its movement through this thin layer must have a surface substantially equivalent to the exit orifice (3) passage surface.

Due to this geometric configuration, the gas flooding the pressure chamber experiences a strong acceleration (changing abruptly both velocity and direction) when crossing the exit hole of the tube and meeting the axial flow of the liquid exiting the tube. Hence, the liquid exiting the tube experiences a violent implosion as a consequence of the intense radial and centripetal component of the gas interacting with it. This produces in the liquid in the exit section of the tube, a high pressure central area and, at the same time, a low pressure close to the inner border of the tube exit. As a consequence, a vorticity pattern is created in the liquid that produces the appearance of violent turbulent unsteady motion in the same exit area of the liquid of the tube. The intense shear stress interaction between both phases, liquid and gas, in the mixing area, together with the emergence of said violent turbulent motions of the liquid separate the liquid very efficiently into small droplets. The mixture of the two phases leaves the pressure chamber through the orifice (3) as a very dense aerosol characterized by having extremely small drops. The drop size distribution created depends basically of:
(a) p0: pressure of the bearer gas inside the pressure chamber at sufficiently large distance from its exit orifice,
(b) the liquid flow rate exiting the tube,
(c) the particular geometry of the device in the surroundings of the orifice (outer shape of the tube, treatment of the surfaces around it and the same exit orifice, diameter of both, etc.) and
(d) the geometry of the orifice borders and the hole of the tube.

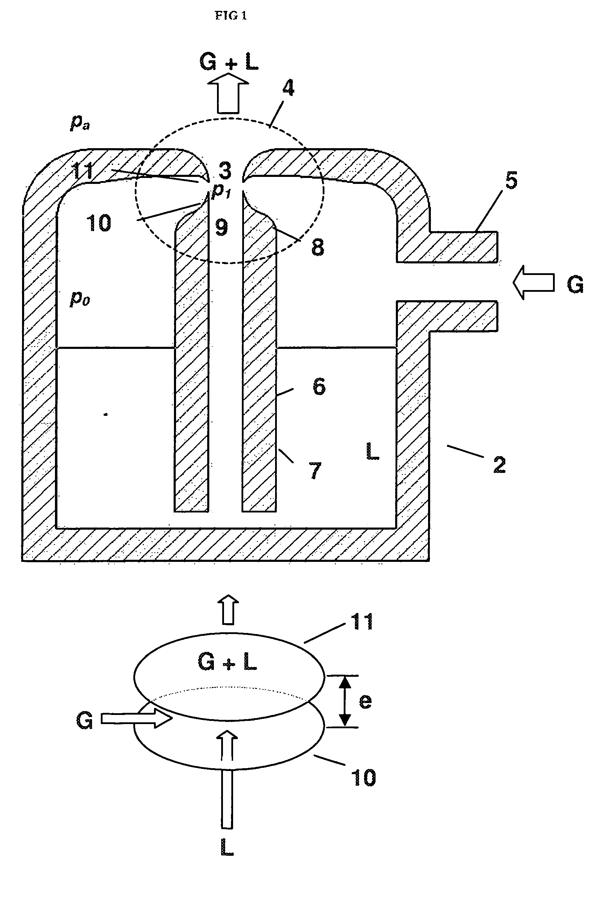

The bearer gas flooding the pressure chamber (see figure 1) experiences a pressure drop from the pressure in said chamber (p0) to the room pressure (pa); the gas reaches room pressure precisely in the exit section of the exit orifice (3) of the chamber. Thus, in the surroundings of the orifice (3) the gas pressure (p1) is higher but close to room pressure. If the exit hole of the tube of liquid is placed close enough to the chamber exit orifice (a necessary event when the gas passage section previously described between the tube and the orifice is equivalent to the exit orifice section) the gas pressure at the exit of the liquid tube will be smaller than the gas pressure in the pressure chamber in areas far enough from the orifice. Hence, if the liquid comes from a container connected to the pressure chamber (according to the *joint pressurization* principle) there is a pressure difference between the free surface of the liquid in the container containing it (p0, pressure chamber pressure) and the exit of the liquid tube (p1, pressure close to room pressure). Therefore, if the inlet of the tube (7) is immersed in the liquid of the container (1), the pressure difference between its ends (7 and 8) will cause the movement of the liquid through the tube, that is, the bearer gas "sucks up" the liquid and drives it to the interaction zone (4), where it is broken up into droplets and is then carried to the exterior of the chamber in the form of a fine aerosol.

With a configuration as described, only a single external energy source is required (pressurized gas) to produce the atomization, an external pumping system for the liquid being superfluous. In this configuration, the liquid flow rate is controlled through three parameters:
(a) the distance from the edge of the tube and the exit orifice of the mixture; that distance controls the mentioned minimal passage section for the diaphragm-flow,
(b)the pressure difference caused by the height difference between the free surface of the liquid in the container and the exit of the tube (in devices small enough the influence of this parameter is negligible); and
(c)the pressure drop caused by the head loss produced during the transport of the liquid from the container to the exit of the tube.

It is easier to control this third parameter because the first one would require modifying the device geometry, and the second changes with time (the height of the free surface decreases as the liquid is consumed). Through the interposition of local head loss, the liquid flow rate can be controlled precisely and, therefore, the characteristics of the aerosol obtained.

The method hereby described is shown to be extremely effective in the production of aerosols and suspensions, because it maximizes the interaction between the bearer and the dispersed phase. The efficiency of the atomization of the AFF, described as the proportion of the total energy introduced in the system (by the bearer gas) that is effectively used to the generation of the dispersed phase surface, reaches values much higher than those reached by other current pneumatic methods.

This geometry originates the following fact, essential for the invention: the pressure in the exit hole of the feeding tube (p1) is comprised between the values of the room pressure (pa) and the integral pressure of the gas and the liquid (p0).

The device and procedure object of the present invention lead to the production in continuous regime of a good quality aerosol with no need to turn to an impulsion gas other than atmospheric air or the steam coming from one single source. A light pressurization is enough to impulse the mixture of fluids and the atomization of the liquid. This ensures a very moderate energetic consumption, making the invention compatible with renewable and self-sufficient energy sources (sun collector, wind power).

The invention can as well be complemented with the following addition: the borders of the orifice and hole (10 and 11), or their nearby surroundings, can present some surface finish (roughness, sawing, undulation) with a typical dimension smaller than the average diameter *dj* of the liquid jet created in the exit orifice. This effect can be chosen among the following or similar effects:
(i) a kind of carving of the borders (10 and 11), such as undulation or sawing in radial and /or azimuthal direction with a wave length inferior to *dj,*
(ii) a carving in the surroundings of the exit orifice border (3) such as undulation or denting with a characteristic length smaller than *dj* or,
(iii) any carving combination of the borders or their surroundings (10 and 11).

Additionally, the height and amplitude of those carvings described above must be bigger than the thickness δ of the viscous boundary layer formed by the gas flow at the surfaces (10 and 11).

The existence of a carving on the borders (10 and 11) or their surroundings originates disturbances in the radial gas flow that absorbe a big amount of kinetic energy of the gas flow during its evolution when it is forced to implode radially on the liquid, interact with it, and change direction to exit through the exit orifice (3). This energy absorption takes place by means of a well known mechanism of flow instability development, that turn into turbulent fluctuations, whose evolution and growth are strongly non linear with time. As a consequence, the kinetic energy of the disturbances caused by said carving is transferred, by the known mechanism of turbulent flows, to the small scale disturbances, which finally break the surface of the liquid and separate it into small droplets.

In short, the particular features of the present invention are:
- In general it is a crossed flow pneumatic device, but contrary to other devices, here a completely perimetric cross-flow is generated, which breaks up the liquid jet; hence, the invention follows the scheme of *violent flow-focusing* (D1): encounter between the axial flow of liquid, intercepted by a perimetrical gas flow, directed radially and centripetally.
- The geometric specification based on the facing position of the correlative edges of the exit hole of the liquid and the exit orifice of the mixture. The small gap between both borders defines a minimal ring-shaped passage section that the gas flow must cross radially to intercept the liquid jet emerging from the feeding tube. It is a *perimetrical cross- flow* (diaphragm-flow) strangulating and breaking up a liquid jet.

- The minimal ring-shaped passage section has a surface of the same order as the exit surface of the liquid.
- For the same available pressure difference between the pressure chamber and the outside, this layout guaranties a maximal speed of the gas in the encounter section because it minimises the friction pressure drop previous to the exit through the ring-shape passage surface: while the pressure drop was caused by the guidance passageways of invention D1. Maximizing the gas speed in the area of interaction with the liquid, the speed differences between the phases are obviously maximized, leading to intense disturbance of the liquid and ensuring optimal energy transference between phases. The high gas speeds represent an essential difference in regard to the flow-focussing procedure.
- The integral pressurization of both phases allows controlling the flow with simple elements causing local head loss inserted in the liquid feeding tube.

### Example of embodiment of the invention

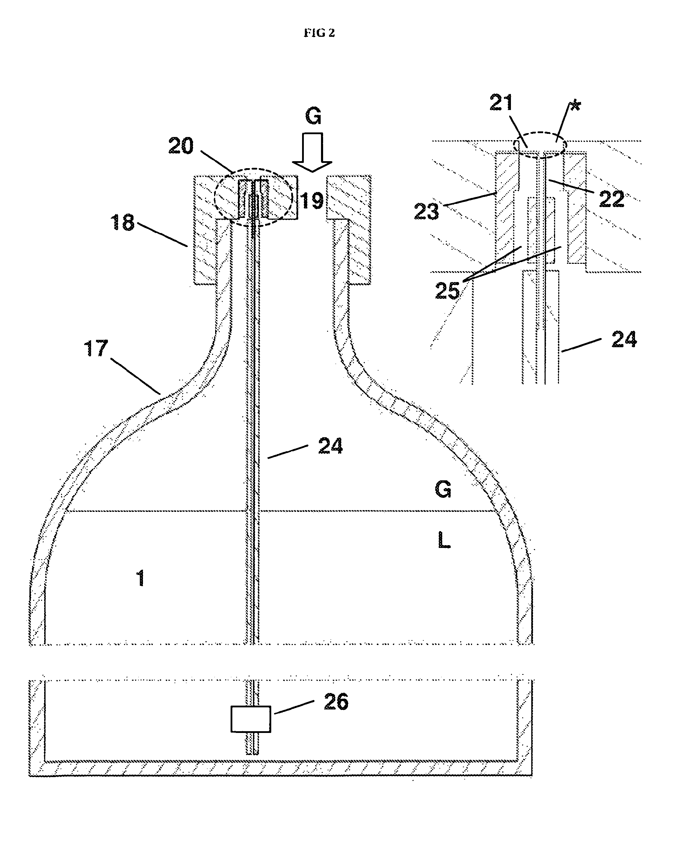

Next the embodiment of a real device is shown where the AFF technology has been successfully integrated (see figure 2, not part of the invention). Said device is made of a two litre capacity plastic bottle (17), in whose opening there is a threaded lid (18), made out of plastic as well. The screw must be leakproof, by means of a sealant or a suitable o-ring. The gas coming from an external pressurization source is introduced through an opening (19) in the lid. In the present execution such pressurization source is an air diaphragm pump consuming 15 watts of power.

Also, there is a cavity (20, see detail) in the lid, where several parts are integrated:
(a) a steel disc (21) of 4 millimetres diameter and 0,1 millimetres thickness with an orifice of 0,4 performed in its centre.
(b)A capillary tubing (22) of steel with an inner diameter of 0,4 millimetres with a straight profile on its tip and placed 0,1 millimetres from the inner plate face (21),
(c)A plastic positioner (23), whose main mission is to ensure that the capillary (22) is correctly facing the orifice plate (21) and at the desired distance from it.

All these parts are pressure fitted, ensuring there are neither relative movements among the parts nor unwanted leaks to the outside. The capillary tubing (22) is connected to the liquid in the bottle by a flexible Tygon ® tube with a 0.3 millimetres of inner diameter and 2,5 millimetres of outer diameter.

The operation of the device is as follows: the gas accesses the bottle (2) through the opening (19) in the lid (18) and pressurizes the interior of the bottle. According to the physical phenomena described previously, said gas impulses the liquid contained in the bottle and forces it to raise through the tube (24) connecting the bottom of the bottle to the higher tip of the capillary (22), while the gas enters the pressure chamber to the mixing zone through one or several gas passage orifices (25) performed on the possitioner (23). According to the physical phenomena described previously, the gas and the liquid are mixed and give rise to an aerosol. In the lower tip of the tube (24), a localized head loss point is set (26), in order to control the flow rate of nebulized liquid.

This localized pressure drop (26) can be, for example, a tube 3 millimetre long, 0,1 millimetre of inner diameter and 1,5 millimetre of outer diameter, introduced at the tip of the Tygon tube.

### Aim of the invention

### General configuration

The device of Fig. 1 is not part of the invention and is used for the atomization of a liquid by means of an impulsion gas or steam (from now on gas (G)) that is pressurized into the mentioned device; both fluids are expelled to the outside as an aerosol or suspension of droplets transported by that gas; such device comprises of a liquid storage chamber (L) (1), contained in a container or pressurized bottle (2) at the pressure (p0) of integral pressurization of the gas and liquid; that container is leakproof and includes an injection inlet (5) that permits the entry of the pressurized gas; the container also has an exit orifice (3) for the mixture gas/liquid; that exit orifice is placed in a mixing area (4) where the two phases gas and liquid are combined, leading to the breakup of the liquid flow and the exit to the outside as an aerosol; the impulsion gas, after entering the container (2) through the injection inlet (5), exits through another exit orifice (3) to the environment; the free liquid surface inside that container (2) is pressed by the impulsion gas to the mixing area through a feeding tube (6), whose aspiration inlet (7) is placed close to the bottom of the bottle (2); the other end of the feeding tube, called nebulizer end (8), comprises of an exit hole (9) whose outer border (10) is approximately facing the inner border (11) of that exit orifice (3); between both facing borders there is a small axial gap (e) that defines a ring-shaped passage section for the impulsion gas (see detail of Fig. 1); that exit orifice (3) is drilled on a wall of that container (2) that is approximately located on a perpendicular plane to the axis of that nebulizer end (8); the gas coming from the pressurized bottle has to cross radially the ring-shape passage section between the inner border (11) of the exit orifice (3) and the outer border (10) of the exit hole (9) to go to the outside through that exit orifice; the ring-shape passage is the minimal section in the radial path of the gas and has a surface of the order of the section of the exit hole; the pressure at the exit hole of the feeding tube (p1) is comprised between the values of outer room pressure (pa) and the integral gas and liquid pressurization pressure (p0).

### Different configurations of the exit orifice and exit hole

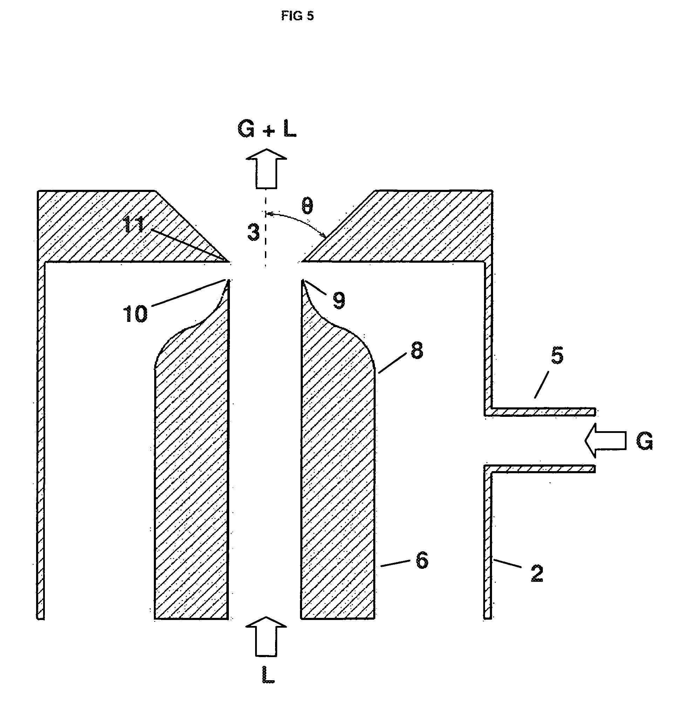

In particular, it is also in the scope of the present invention a design as follows: the outer face of the wall of the container in the surroundings of the exit orifice (3) is carved to create an approximately conic crater (see Figure 5), whose edge coincides with the inner edge (11) of the orifice section in the inner face of said wall. Thus, the orifice walls are not parallel to the axis itself, but they form a certain angle with the orifice axis.

It is also in the scope of the present invention a device for the atomization of a liquid by means of an impulsion gas according to the above mentioned where the orifice and hole edges (10 and 11), or their close surroundings, can present some surface finish effect (roughness, sawing, undulation) of a characteristic size smaller than the average diameter *dj* of the liquid jet flowing through the exit orifice after the liquid exits said exit hole (9), and higher than the thickness of the boundary of the gas at the solid walls.

### Configuration of two chambers with exhaust valve

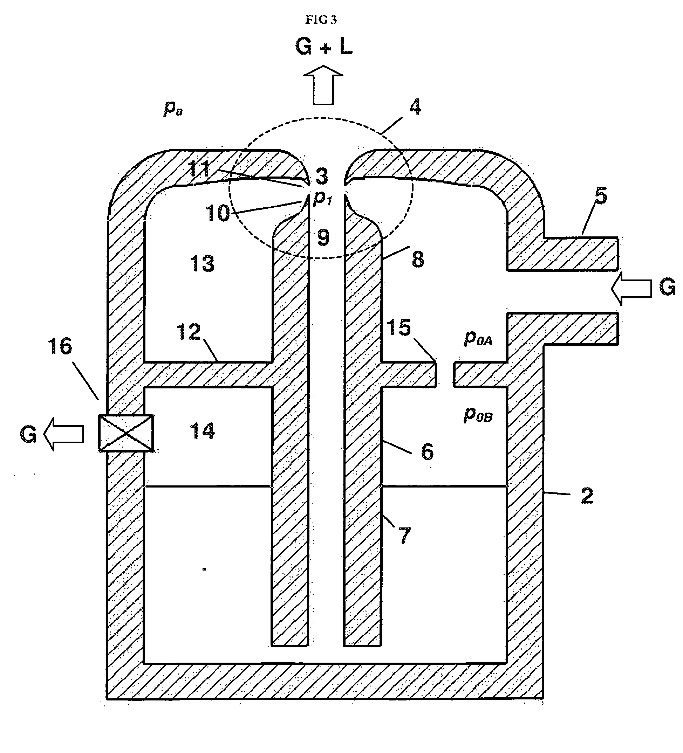

It is also in the scope of this invention a device with a configuration (see Figure 3) alternative to the general configuration described previously. The new features of this configuration are: *(i)* the gas pressure chamber is sub-divided by a partition wall (12) into two new ones, higher (13) and lower (14), that are connected through a connection port (15); *(ii)* all the liquid is contained in the lower chamber, where there is a gas purge valve (16) opening to the environment. This configuration presents some advantages compared to the configuration described previously, in particular providing a stronger control on the nebulization and the integration of a purge or cleaning procedure in the same device.

The performance of this particular configuration is based on the pressure difference between the higher chamber (13) at pressure p0A, and the lower chamber (14), at pressure p0B. This pressure difference is due to a pressure drop experienced by the gas when it crosses the connection port (15) existing on the wall (12). According to the opening status of the valve (16) we can see two different performance conditions:
(a) Nebulization (closed valve): in this case the valve (16) is closed; most of gas exits to the outside through the exit orifice (3) and a small part crosses the connection port (15) and occupies the free volume that has appeared as a consequence of the liquid consumed in the nebulization. In most of the cases (provided that the fluid density of the bearer phase is much lower than that of the fluid in the dispersed phase, a necessary event when they are gas and liquid respectively) the volumetric gas flow rate exiting to the outside through the orifice (3) during the nebulization is much higher than the volumetric nebulized liquid flow rate. Because of that, the gas flow accessing the lower chamber (14) through the connection port (15) is very small, thus making the pressure drop negligible; in this case, the pressures of the higher and lower chambers are hence practically identical (p0A - p0B). Thus, the liquid contained in the lower chamber is pressurized at initial gas pressure and the nebulization takes place as it had been described previously in the general case where there was one only chamber with no valve.
(b) *Cleaning or purge* (open valve): in this case the valve (16) is open. Designing and dimensioning adequately the connection port (15) and the valve (16), it can easily be achieved that the gas pressure drop when crossing the open valve is negligible compared to the pressure drop experienced when crossing the connection port (15). Thus, the lower chamber (that is connected to the exterior) reaches a pressure very close to the exterior pressure (p0B ~ pa). In this case, the volumetric gas flow rate accessing the lower chamber (14) through the connection port (15) is not negligible, neither is the pressure drop caused in the path. Dimensioning the connection port (15) so that the pressure drop at its crossing is larger than the pressure drop to reach the exit hole (9) of the tube (6), (that is, such that p0A - p0B > p0A - p1) a differential pressure between the tube ends appear (p0B < p1) forcing the liquid exiting in it to revert its flow and return to the deposit (1). Thus, when opening the valve two effects are achieved: shutting abruptly the nebulization (permitting a precise control on it) and cleaning the liquid tube (leading to multiple advantages which will be explained further on).
(c) *Controlled nebulization* (valve in intermediate position): in this case the valve is in an intermediate position between the states "closed" and "open". So, starting from a gas supply at constant pressure p0A, the pressure of the lower chamber (14) p0B can be controlled by means of the opening of the valve (16). Since that p0B pressure is the liquid impulsion pressure, by means of a controlled partial opening of the valve characteristics in the aerosol can be controlled such as flow rate or the size of the produced drop. The activation of the valve can be controlled both automatic or manually (i.e. an aerograph lever), allowing the user in this last case to have certain "sensibility" on the produced aerosol.

Therefore the configuration with two chambers and exhaust valve allows controlling with precision the duration of the nebulization (start and stop), its characteristics and it also integrates a cleaning or purges functionality.

In relation to the other described elements, it must be emphasized that there are no restrictions neither in the nature and characteristics of the wall (12) that separates the higher chamber (13) from the lower (14) nor in the connection port (15) that joins both chambers. They can be as simple as a plate with a single orifice, one plate with multiple orifices located in any configuration (reticular, circular, etc.), a filter of a determined pore size or any other separation media allowing to achieve the desired pressure drop. Though the simplest configuration consists of fix and immobile structural elements, this does not avoid the usage of any element or assembly of elements with one or several mobile parts driven manually or automatically (i.e. valves controlled electronically from the outside). Any solution is possible as long as both chambers are separated in a way that produces the desired pressure drop of the gas when it crosses the connection port (15).

Given the relative position of the two chambers or compartments, being all the liquid contained in the lower compartment, we need to restrict in this configuration the inclination of the bottle while it is in use. As an alternative solution, it is possible to design the connection port between the two chambers with a device that prevents the passage of liquid from the lower to the higher chamber.

### Configuration with three chambers and exhaust valve

This configuration (Fig. 4) is a variation of the previous one, where the higher compartment (13) is sub-divided into two receptacles: a pressurization receptacle (13a) connected directly to the pressurized gas inlet (5) and an impulsion receptacle (13b), surrounding the mixing area; both receptacles are connected to each other through a second connection port (15a) that creates a pressure drop in the gas flow between both receptacles. The aim of this division in three chambers is to increase the control options of the device, especially in regard to the pressure distribution in the mixing area (Fig. 1, detail).

### Configuration with removable liquid container

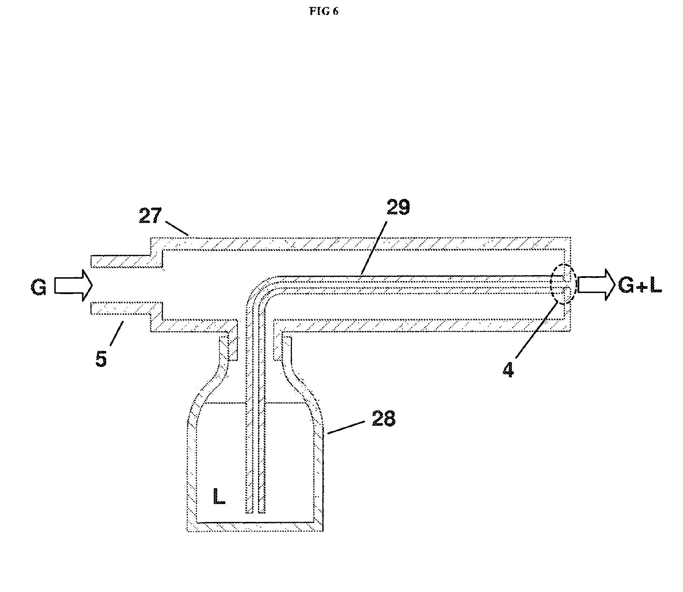

The device of Fig. 6 in which the pressurized container comprises two parts assembled to each other is not part of the invention. The first of them is a chamber (27) full with gas where the mixing area for the gas and liquid is integrated. The second part is a container for the liquid (28) which is assembled to the previous one by any joining mean ensuring that it remains fixed and leakproof (thread, pressure adjustment, etc.). The gas is introduced in the device through a inlet (5), which can be integrated both in the chamber (27) and in the liquid container (28). The liquid container can be used again after cleaning it or be disposable capsules. In the usual case where the liquid is atomized with a gas (and in general, whenever the bearer phase is less dense than the dispersed phase), the gas entry to the container can even be done under the free surface of the liquid, so that the gas will produce bubbles in the liquid that will later raise and pressurize the area above the free surface of the liquid. This pressurized gas impulses the liquid from the container (28) to the mixing area (4) through a tube for liquid transport (29).

The container (28) has a valve and is separated from the chamber (27) by means of a wall where there is a connection port such as it was explained before for the general device with two chambers and an exhaust valve. Thus, in this device all advantages of the two chambers with exhaust valve configuration add up (cleanliness possibility, nebulization control, etc.) and the possibility to disassemble the liquid container as desired for cleaning or charging purposes.

### Pressure drop control

In another version of the invention, the feeding tube (6) incorporates a pressure drop control. This pressure drop control, which can be included in any of the described devices, may consist of a simple fix obstacle to the passage of liquid (i.e. a stretch of tube of small diameter, a filter with the adequate pore size, a frit, etc.) or else it can be an element with variable pressure drop (i.e. a valve controlled externally).

### Impulsion with steam

In this version of the invention the pressurized gas used to produce the atomization is a steam (V) obtained from a vaporizable liquid (Lv). Without this meaning a restriction to the liquid, that vaporizable liquid can be chosen among the following substances or their combinations: water CFC's, alcohols, ketones, ethers, esters, paraffins, alkanes, cycloparaffins, naphthenes or cycloalkanes or aromatic hydrocarbon, olefins, alkenes and other non saturated hydrocarbons.

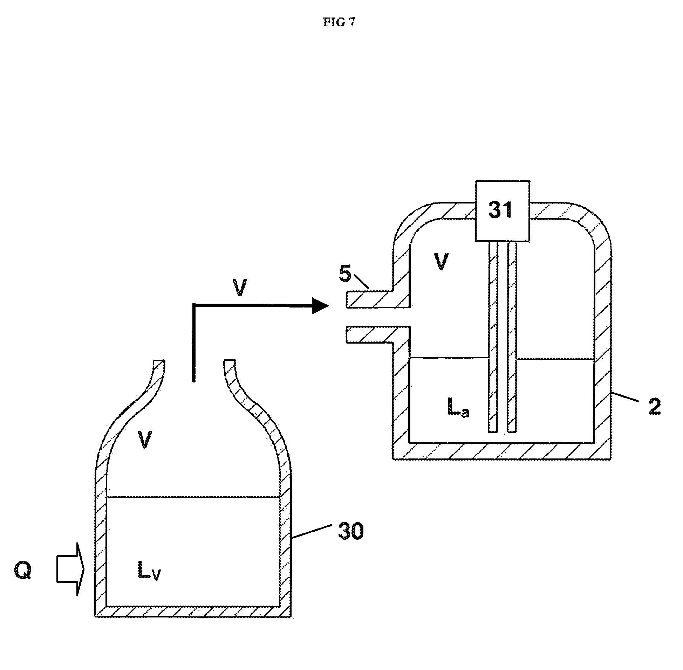

In general, the vaporization of the vaporizable liquid is done by applying heat. Applying heat can be done externally, in a container independent from the device body (Fig. 7), where the liquid to be atomized (La) is stored separately.

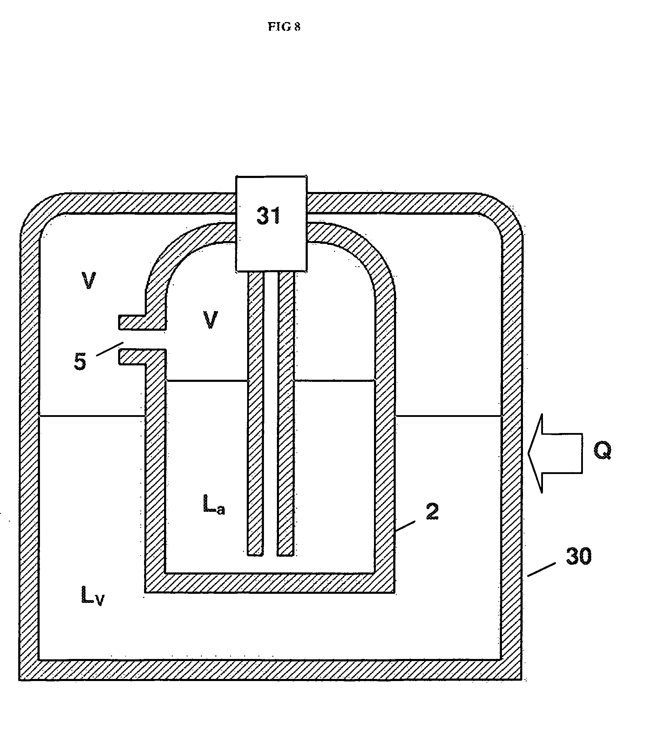

As an option, both liquids, atomizable (La) and vaporizable (Lv), can be stored together in the device (Fig. 8) using an adequate separation; then applying heat will heat up both liquid to be vaporized and liquid to be atomized. Typically, the bottle or pressurized container (2) is surrounded (as a *Thermos* bottle) with a layer for vaporizable liquid storage. The common heating of both liquids vaporizes though only one of them, the one contained in the outer layer that being already in vapour phase, enters the interior of the pressure chamber through the inlet (5). This method requires the boiling point of the vaporizable liquid to be sufficiently smaller than the boiling point of the liquid to be atomized.

In this configuration, generally the temperature of the two liquid phases previous to their vaporization or atomization is approximately the same.
In this configuration, the caloric power supplied to keep the temperature common to both liquid phases can be used as control parameter of the pressure and the vapour flow rate introduced under pressure through the injection inlet (5).

An alternative option involves using as raw material in the device a solid to be atomized. For that it is required to achieve its phase change, generally by heat application. The solid can be introduced as grain, powder, bars or lumps. The most favourable configuration is achieved when the solid phase to be turned into atomizable liquid and the vaporizable liquid to be turned into impulsion vapour are stored together with the appropriate separation (of the double wall thermal bottle type), and they are heated simultaneously to a temperature almost -common to both of them to ensure the production of the working conditions.

The caloric power applied to keep a common temperature in both liquid phases is also used as a control parameter of the surface tension and the viscosity of that liquid to be atomized. These properties, in fact, are essential for controlling the drop size.

### Application

Finally, the invention includes a procedure for the atomization of a liquid by means of an impulsion gas according to the devices described in the paragraphs above.

This procedure can be use for different aims: in one of the foreseen cases, the aerosol or drop suspension produced is used for humidification or air conditioning of spaces, both interior and exterior.

In the case of mixing the liquid to be atomized with some additives, the aerosol can be used for air conditioning, air freshening, balsamic substances dispersion, disinsecting, biologic control of airborne infectious transmission illnesses and other applications where atmospheric air constitutes the basic transport vehicle of droplets or their rests (after the evaporation of the liquid or solvent) to the target final areas, that can be the respiratory system of any living being, its outer skin, or its eyes.
When the liquid contains pigments, polymers, monomers or other substances such as paintings, polishes, ceramic or metallic particles, oils or outer skins of any nature, those target final areas can also be of any kind that is wanted to be treated by deposing those mentioned substances on those surfaces.

In another foreseen application, the aerosol or drop suspension produced is used for food production.

In another application explicitly included, the liquid is a fuel and the gas is a comburent, and the aerosol or droplet suspension gas produced is used as mixture in an internal combustion engine.

It is as well foreseen another application in which the aerosol is used to refrigerate the machining area and the tool in any machining operation of a metallic, polymeric or ceramic material.

In an additional foreseen application, the aerosol is used as volumetric collecting mean for powder, particles of all nature or molecules suspended in the media where the aerosol is dispersed.

### Description of the figures

Figure 1: General drawing of the AFF device with joint pressurization.
   In this drawing the gas (G) is introduced into the pressurized container (2) through the inlet (5). The pressure of the gas impulses the liquid (L) contained in the storage chamber (1) and drives it through the tube (6) up to the mixing area (4) where it interacts with the gas producing an aerosol that exits to the outside through the exit orifice (3).
Figure 1 (inferior detail): main geometry of the invention in the mixing area (4) including the inlets of liquid and gas, the exit of the mixture and the edges 10 and 11. The liquid flow is intercepted radially in perimetrical flow pattern. This is therefore a perimetrical cross-flow where a gas diaphragm-flow strangulates a liquid jet.
Figure 2: Drawing of manufactured device.
   In this drawing the gas (G) enters a plastic bottle (17) through the opening (19) and impulses the liquid (L) contained in the storage chamber (1) to the mixing area along a feeding tube (24) firstly and the capillary tubing (22) afterwards. In this area the liquid interacts with the gas producing an aerosol that exits to the outside through the exiting orifice on the plate (21). The detail (*) shows a mixing area according to the description in figure 1.
Figure 3: Drawing of device with two chambers and an exhaust valve.
   In this figure the gas enters the container (2) through the inlet (5) and pressurizes the higher chamber (13) and the lower chamber (14) to the pressures p0A and poB. These pressures are determined by the pressure drop experienced by the gas when crossing the connection port (15) and the exhaust valve (16). In case the valve is closed, the pressure in the lower chamber impulses the liquid through the tube (6) to the mixing area (4) where it interacts with the gas and forms the aerosol exiting to the outside through the exit orifice (3). On the contrary, if the valve is open, the introduced gas exits to the outside through the exit orifice (3) and the valve itself (16); the pressures created in the higher and lower chambers produce a pressure difference between the superior and inferior ends of the tube (6), so that the liquid contained in it circulates back to the storage chamber (1).
Figure 4: Drawing of device with three chambers.
   This drawing is similar to the device described in the previous figure, but the high compartment is sub-divided into two containers (13a and 13b), separated from each other by a second connection port (15a). The pressure drop associated to this second port allows adjusting the pressure (p0C) in the higher chamber and therefore, the pressure (p1) in the mixing area (4).
Figure 5: Particular configuration in which the outer walls of the orifice form an angle θ with its axis.
Figure 6: Drawing of a device characterized by a joint pressurization chamber where the gas and liquid are placed, consisting of two removable parts (27, 28), all of the liquid being contained in one of them (28). The gas is introduced through the inlet (5) and impulses the liquid from the liquid storage chamber (1) to the mixing area (4) through the transport tube (29).
Figure 7: Drawing of device with external vaporizer.
   The vaporizable liquid (Lv) contained in the vaporizable liquid chamber (30) is heated by supplying an amount of heat (Q). The produced vapour (V) is driven to the inlet (5) and accesses the inside of the bottle (2). The vapour is used to impulse the atomizable liquid (La) to the mixing head (31), where it is mixed with the vapour producing the aerosol.
Figure 8: Drawing of device with joint vaporizer.
   The supplied heat (Q) is used on the one hand to heat the liquid to be vaporized (Lv) and to play the role of an impulsion vapour (V) and, at the same time, to heat the liquid to be atomized (La).
   The impulsion vapour and the impulsed liquid reach the mixing head (31) and they are mixed producing the aerosol. As an option, the atomizable liquid (La) is in solid phase before being heated.

### Description of the figure labels

1. Liquid storage chamber
2. Container or pressurized bottle.
3. External exit orifice of the mixture gas / liquid
4. Mixing area
5. Injection inlet of the gas in the bottle
6. Feeding tube of the liquid
7. Aspiration inlet of the feeding tube
8. Nebulizer end of the feeding tube
9. Exit hole of the liquid tube
10. Outer edge of the exit orifice in the nebulizer end of the feeding tube
11. Inner edge of the exit orifice located in the container wall; between both edges, positioned facing each other and with a small interposed axial interval, a passageway is created for the transversal movement of the gas to the exit
12. Wall dividing the container into two pressure chambers, lower and higher
13. Higher chamber in the two-chambers configuration;
   a. Pressurization container connected directly to the inlet of entrance of the gas under pressure (5) in the three-chambers configuration
   b. Impulsion container surrounding all the mixing area in the three-chambers configuration.
14. Lower chamber; containing all of the liquid: this fact restricts the operation angle of the device or, alternatively, forces to design the connection port in such a way that the passage of liquid from the lower chamber to the higher chamber is avoided.
15. Connection port between higher and lower chambers a. Second connection port between pressurization and the impulsion container in the configuration with three chambers
16. Gas passage valve between the lower chamber and the environment
17. Plastic bottle
18. Threaded lid in the bottle mouth
19. Opening located in the lid in order to give passage to the gas from an external source
20. Cavity lodging the lid
21. Steel plate with a thickness of 0,1 millimetres and a centered orifice of 0,4 mm
22. Steel capillary tubing with 0,4 millimetres of inner diameter
23. Positioner to ensure the relative position between the plate and the capillary tubing
24. Feeding tube for the liquid in the manufactured configuration described
25. Gas passage orifice from the pressure chamber to the mixing area
26. Element to produce a located and controlled pressure drop
27. Pressure chamber where the mixing area is located
28. Removable container containing the liquid
29. Liquid transport tube
30. Chamber of the liquid to be vaporized
31. Mixing head

p0 feeding tube upstream pressure: it is the joint pressurization pressure (p0) for gas and liquid
pa external room pressure (pa)
p1 pressure at the exit of the feeding tube exit hole (p1)
p0A higher chamber pressure in the two-chambers and exhaust valve configuration
p0B lower chamber pressure in the two-chambers and exhaust valve configuration; pressure in the intermediate chamber connected with the bottle inlet in the three-chambers and exhaust valve configuration
p0C pressure in the higher chamber in contact with the mixing area in the three-chambers and exhaust valve configuration
L liquid
G gas
V vapour
La Liquid to be atomized. As an option it can be a solid (in lumps, grain, powder, pills) melting when heated.
Lv Liquid to be vaporized.
Q Supplied heat.

## Claims

1. - Device for the atomization of a liquid by means of an impulsion gas or steam that is pressurized into said device, both fluids being expelled to the outside as an aerosol or droplet suspension transported by said gas or steam, the device consisting of a liquid storage chamber (1), contained in a container or pressurized bottle (2) under joint gas and liquid pressurization pressure (p0); said container (2) being leak proof and includeing an injection inlet (5) that allows the introduction of the pressurized gas or steam and an external exit orifice (3) for the gas/liquid mixture; the exit orifice (3) being located in a mixing area (4) where the combination of both gas and liquid phases takes place, as well as the breakup of the liquid flow and its exit to the outside as an aerosol; the impulsion gas or steam, after entering the container (2) through the injection inlet (5), exiting through said exit orifice (3) to the environment; the free surface of the liquid inside the container (2) being pressed by the impulsion gas or steam, so that the liquid is impulsed to the mixing area through a feeding tube (6), whose aspiration inlet (7) is located close bottom of the container (2); the other end of the feeding tube, the so-called nebulizer end (8), consisting of an exit hole (9) whose outer edge (10) is approximately facing the inner edge (11) of the exit orifice (3), thereby forming between both facing edges a short axial difference that defines a ring-shaped passage section for the impulsion gas or steam; said exit orifice (3) being drilled in a wall of the container (2) and approximately located in a plane perpendicular to the axis of the nebulizer end (8); the gas or steam coming from the pressurized container crossing radially said ring-shaped section comprised between the inner edge (11) of the exit orifice (3) and the outer edge (10) of the exit hole (9) before exiting to the outside through the exit orifice (3), said ring-shaped passage section being the minimal section in the radial trajectory of the gas or steam and its area is of the order of the area of the exit orifice (3); the pressure (p1) in the exit hole of the feeding tube (6) being comprised between the values of the external room pressure (pa) and the joint gas and liquid pressurization pressure (p0), **characterized in that** the pressurized container (2) includes a partition wall (12) that sub-divides it into two compartments; all the liquid contained in the lower compartment (14), where there is a gas all the liquid being contained in the lower compartment (14), where there is a gas purge valve (16) opening to the environment, the injection inlet (5) of the pressurized gas or steam being located in the higher compartment (13); both compartments being connected to each other through a connection port (15) located in the partition wall (12); said port (15) determineing a pressure drop in the gas or steam flowing between the two compartments; the feeding tube (6) crosseing said partition wall (12), in such a way that the passageway through the partition wall achieved thereby is watertight; three operational phases being distinguished: nebulization, with closed valve (16); cleaning or purge, with open valve (16) and reverted as flow in the feeding tube (6), from the exit hole (9) to the aspiration inlet (7); controlled nebulization, with partially closed valve (16) position.

2. -Device for the atomization of a liquid by means of an impulsion gas according to claim 1, **characterized in that** the higher compartment (13) is sub-divided into two receptacles: one pressurization receptacle (13a) connected directly to the pressurized gas entry inlet (5) and an impulsion receptacle (13b), that surrounds said mixing area, both receptacles being connected to each other through a second connection port (15a) that creates a pressure drop in the gas flow between the two receptacles.

3. - Device for the atomization of a liquid by means of an impulsion gas according to claims 1 and 2, **characterized in that** the outer face of the container (2) wall in the surroundings of the exit orifice (3) is carved to create an approximately conic crater, whose edge coincides with the inner edge (11) of the orifice section in the inner face of said wall.

4. - Device for the atomization of a liquid by means of an impulsion gas according to the claims 1 to 3 **characterized in that** the orifice and hole edges (10 and 11), or their close surroundings, may present some surface finish effect like roughness, sawing, undulation of a characteristic size smaller than the average diameter dj of the liquid jet flowing through the exit orifice after the liquid exits said exit hole (9), and higher than the thickness of the boundary layer of the gas at the solid walls.

5. - Device for the atomization of a liquid by means of an impulsion gas according to the claims 1 to 4, **characterized in that** the pressurized container (2) comprises two parts connected to each other, all the liquid being contained in one of the parts, and the liquid container being a removable part for cleaning or charging purposes.

6. - Device for the atomization of a liquid by means of an impulsion gas according to claim 5, with the characteristic that the two parts that compose the container are joined with a thread, pressure or by means of a closure, adjustment or external fixation element.

7. - Device for the atomization of a liquid by means of an impulsion gas according to claims 1 to 6, **characterized in that** the feeding tube (6) incorporates a pressure drop control.

8. - Device for the atomization of a liquid by means of an impulsion gas according to claims 1 to 7, **characterized in that** the pressurized gas is a vapour obtained from a vaporizable liquid or solid.

9. - Device for the atomization of a liquid by means of an impulsion vapour according to claim 8, **characterized in that** the vaporizable liquid is chosen among the following substances or a combination of them: water, alcohols, chlorofluorocarbons (CFC's), ketones, ethers, esters, paraffin, alkanes, cycloparaffins, naphthenes or cycloalkanes or aromatic hydrocarbon, olefins, alkenes and other non saturated hydrocarbons.

10. - Device for the atomization of a liquid by means of an impulsion vapour according to claims 8 to 9, **characterized in that** the vaporization of said vaporizable liquid or solid is made by means of heat supply.

11. - Device for the atomization of a liquid by means of an impulsion vapour according to claim 10, **characterized in that** both atomizable and vaporizable substances are stored in the device with an appropriate separation; and that heat supply heats at the same time the liquid or solid to be vaporized and the liquid to be atomized.

12. - Device for the atomization of a liquid by means of an impulsion vapour according to claim 11, **characterized in that** the temperature of both liquid phases, atomizable and vaporizable, previously to their vaporization or atomization, is approximately the same.

13. - Device for the atomization of a liquid by means of an impulsion vapour according to claim 12, **characterized in that** the caloric power supplied to keep the temperature common to both liquid phases is used as control parameter of the pressure and the vapour flow rate inserted under pressure through the injection inlet (5).

14. - Device for the atomization of a liquid by means of an impulsion vapour according to claims 8 to 13, **characterized in that** the liquid to be atomized is in a solid phase, and caloric energy is locally supplied to produce the fusion of the solid phase.

15. - Device for the atomization of a liquid by means of an impulsion vapour according to claim 14, **characterized in that** the solid phase meant to become atomizable liquid and the liquid or vaporizable solid meant to become impulsion vapour are stored together with an appropriate partition wall and are heated simultaneously to a temperature approximately common to both to ensure the generation of the operation conditions.

16. - Device for the atomization of a liquid by means of an impulsion vapour according to claim 15, **characterized in that** the caloric power supplied to keep a common temperature for both liquid phases is used as a control parameter for the surface tension and the viscosity of the liquid to be atomized.

17. - Procedure for the atomization of a liquid by means of an impulsion gas according to the device of claims 1 to 16.

18. - Procedure for the atomization of a liquid by means of an impulsion gas according to claim 17, **characterized in that** the aerosol or droplet suspension generated is used for the humidification or acclimatization of both interior and exterior spaces.

19. - Procedure for the atomization of a liquid by means of an impulsion gas according to claim 17, **characterized in that** the liquid tobe atomized contains additives aimed at air acclimatization, air freshening, dispersion of balsamic substances, disinsectation, biologiccontrol of airborne infectious diseases and other applications where atmospheric air constitutes the basic transport vehicle of droplets or their remnants after the evaporation of the liquid or solvent to the target final areas that can be the respiratory system of any living being, its outer skin, or its eyes.

20. - Procedure for the atomization of a liquid by means of an impulsion gas according to claim 17, **characterized in that** the aerosol or droplet suspension generated is used for food production.

21. - Procedure for the atomization of a liquid by means of an impulsion gas according to claim 17, **characterized in that** the liquid is a fuel and the gas is a comburent, and the aerosol gas or droplet suspension is as mixture in an internal combustion engine.

22. - Procedure for the atomization of a liquid by means of an impulsion gas according to claim 17, **characterized in that** the liquid is a painting, polish or skin protection, and the aerosol or droplet suspension generated is used for the application of said painting, polish or skin protection on a surface located outdoors.

23. - Procedure for the atomization of a liquid by means of an impulsion gas according to claim 17, **characterized in that** the liquid is a sample for elemental analysis by means of atomic spectrometry techniques.

24. - Procedure for the atomization of a liquid by means of an impulsion gas according to claim 17, **characterized in that** the liquid is a pesticide, insecticide or any other substance used in the agricultural sector for cultivation treatment.

25. - Procedure for the atomization of a liquid by means of an impulsion gas according to claim 17, **characterized in that** the aerosol or droplet suspension is used to refrigerate the machining area and the tool in any machining operation on a metallic, polymeric or ceramic material.

26. - Procedure for the atomization of a liquid by means of an impulsion gas according to claim 17, **characterized in that** the aerosol is used as means for the volumetric collection of powder, particles of all nature, or molecules suspended in the environment where the aerosol is dispersed.

## Patentansprüche

1. Vorrichtung zur Zerstäubung einer Flüssigkeit durch den Druck eines in die Vorrichtung hineingepressten antreibenden Gases oder Dampfes unter Ausstoß beider Flüssigkeiten nach außen in zerstäubter Form oder als Schwebetröpfchen, die von den erwähnten Gasen oder Dämpfen transportiert werden, wobei die Vorrichtung eine Flüssigkeitsspeicherkammer (1) umfasst, die in einem Behälter oder in einer unter Druck stehenden Flasche (2) unter einem gemeinsamen Gas- und Flüssigkeitsdruck (p0) untergebracht ist und der Behälter (2) dicht (frei von Leckagen) ist, zum Eintritt des unter Druck stehenden Gases oder Dampfes über einen Einspritzeinlass (5) verfügt und außerdem eine externe Auslassöffnung (3) für die Gas-Flüssigkeits-Mischung besitzt, die in der Mischzone der Gas- und Flüssigphase (4) angebracht ist, in der auch der Flüssigkeitsstrom endet und als Sprühnebel nach außen austritt, wobei das antreibende Gas oder der antreibende Dampf nach dem Eintritt durch den Einspritzeinlass (5) in den Behälter (2) durch die Auslassöffnung (3) nach außen entweicht, und die freie Flüssigkeitsoberfläche im Behälter (2) durch das antreibende Gas oder den Dampf derart gedrückt wird, dass die Flüssigkeit in die Mischzone strömt und dabei ein Zufuhrrohr (6) durchfließt, dessen Ansaugöffnung (7) nahe am Boden des Behälters (2) angebracht ist, während das andere Ende des Zufuhrrohres, das sogenannte Zerstäuberende (8), eine Austrittsbohrung (9) besitzt, deren Außenkante (10) etwa der Innenkante (11) der Auslassöffnung (3) gegenüberliegt und somit ein kurzer axialer Abstand zwischen den beiden gegenüberliegenden Kanten besteht, der einen ringförmigen Durchflussquerschnitt für das antreibende Gas bzw. den antreibenden Dampf bildet, wobei die Auslassöffnung (3) in eine Wand des Behälters (2) gebohrt ist und näherungsweise in einer Ebene liegt, die senkrecht zur Achse des Zerstäuberendes (8) steht, womit das Gas oder der Dampf aus dem unter Druck stehenden Behälter den genannten ringförmigen Querschnitt, der zwischen der Innenkante (11) der Auslassöffnung (3) und der Außenkante (10) der Austrittsbohrung (9) gegeben ist, in radialer Richtung durchquert, bevor es/er durch die Auslassöffnung (3) austritt, wobei der eben erwähnte ringförmige Durchflussquerschnitt die minimale Querschnittsfläche auf der radialen Wegstrecke (Trajektorie) des Gases oder Dampfes darstellt und eine Fläche von der Größenordnung der Auslassöffnung (3) besitzt und der Druck (p1) in der Austrittsbohrung des Zufuhrrohres (6) zwischen dem Außendruck (pa) und dem gemeinsamen Flüssigkeitsdruck (p0) liegt, **dadurch gekennzeichnet, dass** der unter Druck stehende Behälter (2) mit einer Trennwand (12) versehen ist, die den Behälter derart in zwei Abschnitte unterteilt, dass sich die gesamte Flüssigkeitsmenge im unteren Abschnitt (14) befindet, der über ein Ablassventil (16) verfügt, das den Weg nach außen freigibt, während der Einspritzeinlass (5) des unter Druck stehenden Gases oder Dampfes im oberen Abschnitt (13) angebracht ist und beide Abschnitte durch eine in der Trennwand (12) eingelassene Öffnung (15) miteinander verbunden sind, die einen Druckabfall des strömenden Gases oder Dampfes zwischen den beiden Abschnitten vorgibt und das Zufuhrrohr (6) die genannte Unterteilungswand (12) derart durchstößt, dass ein wasserdichter Weg durch diese Wand gegeben ist; man unterscheidet drei Betriebsphasen: erstens die Zerstäubung mit geschlossenem Ventil (16), zweitens den Reinigung- oder Ablassbetrieb bei geöffnetem Ventil (16) mit umgekehrtem Gasfluss von der Austrittsbohrung (9) durch das Zufuhrrohr (6) zur Ansaugöffnung (7) und drittens die kontrollierte Zerstäubung mit teilweise geschlossenem Ventil (16).

2. Vorrichtung zur Zerstäubung einer Flüssigkeit durch den Druck eines antreibenden Gases nach Anspruch 1, **dadurch gekennzeichnet, dass** der obere Abschnitt (13) in die beiden folgenden Behältnisse unterteilt ist: ein Behältnis zur Druckbeaufschlagung (13a), das direkt mit dem Einlass (5) des unter Druck stehenden Gases verbunden ist, und ein Behältnis zur Druckeinwirkung (13b), das um die erwähnte Mischzone herum verläuft, wobei beide Behälter durch eine zweite Öffnung (15a) miteinander verbunden sind, die einen Druckabfall des strömenden Gases zwischen den beiden Behältern hervorruft.

3. Vorrichtung zur Zerstäubung einer Flüssigkeit durch ein antreibendes Gas nach den Ansprüchen 1 und 2, **dadurch gekennzeichnet, dass** in die Wandaußenseite des Behälters (2) in der Nähe der Auslassöffnung (3) ein annähernd konisch geformter Krater eingelassen ist, dessen Kante mit der Innenkante (11) der Öffnung an der Innenseite der genannten Wand übereinstimmt.

4. Vorrichtung zur Zerstäubung einer Flüssigkeit durch ein antreibendes Gas nach den Ansprüchen 1 bis 3, **dadurch gekennzeichnet, dass** an den Kanten (10 und 11) der Öffnung und der Bohrung oder in ihren näheren Umgebungen bestimmte Oberflächeneigenschaften wie Rauigkeit, Zahnung oder Wellenformen gegeben sind, deren charakteristische Größen einerseits kleiner sind als der mittlere Durchmesser *dj* des Flüssigkeitsstroms, der nach Austritt der Flüssigkeit durch die genannte Austrittsbohrung (9) auch die Auslassöffnung durchfließt, die aber andererseits größer sind als die Dicke der Grenzschicht des Gases an den festen Wänden.

5. Vorrichtung zur Zerstäubung einer Flüssigkeit durch ein antreibendes Gas nach den Ansprüchen 1 bis 4, **dadurch gekennzeichnet, dass** der unter Druck stehende Behälter (2) zwei miteinander verbundene Abschnitte umfasst, die gesamte Flüssigkeitsmenge sich in einem dieser beiden Abschnitte befindet und der Flüssigkeitsbehälter zur Reinigung abnehmbar ist.

6. Vorrichtung zur Zerstäubung einer Flüssigkeit durch ein antreibendes Gas nach Anspruch 5, **dadurch gekennzeichnet, dass** die beiden Teile, die den Behälter bilden, durch ein Gewinde, durch Druck oder mit einem externen Verschluss-, Einstellungs- oder Befestigungselement miteinander verbunden sind.

7. Vorrichtung zur Zerstäubung einer Flüssigkeit durch ein antreibendes Gas nach den Ansprüchen 1 bis 6, **dadurch gekennzeichnet, dass** das Zufuhrrohr (6) eine Steuerung für den Druckabfall umfasst.

8. Vorrichtung zur Zerstäubung einer Flüssigkeit durch ein antreibendes Gas nach den Ansprüchen 1 bis 7, **dadurch gekennzeichnet, dass** das unter Druck gesetzte Gas ein Dampf ist, den man aus einem verdampfbaren Stoff - Flüssigkeit oder Festkörper - erhält.

9. Vorrichtung zur Zerstäubung einer Flüssigkeit durch den Druck eines antreibenden Dampfes nach Anspruch 8, **dadurch gekennzeichnet, dass** es sich bei der verdampfbaren Flüssigkeit um einen Stoff aus der folgenden Liste oder eine Kombination dieser Stoffe handelt: Wasser, Alkohole, Fluor-Chlor-Kohlenwasserstoffe (FCKWs), Ketone, Ether, Ester, Paraffin, Alkanes, Zykloparaffine, Zykloalkane (Naphthene) oder aromatische Kohlenwasserstoffe, Olefine, Alkene und andere ungesättigte Kohlenwasserstoffe.

10. Vorrichtung zur Zerstäubung einer Flüssigkeit durch einen antreibenden Dampf nach den Ansprüchen 8 und 9, **dadurch gekennzeichnet, dass** das Verdampfen des eben genannten verdampfbaren Stoffes - Festkörper oder Flüssigkeit - mithilfe von Wärmezufuhr geschieht.

11. Vorrichtung zur Zerstäubung einer Flüssigkeit durch einen antreibenden Dampf nach Anspruch 10, **dadurch gekennzeichnet, dass** man den zu zerstäubenden und den zu verdampfenden Stoff angemessen getrennt in der Vorrichtung aufbewahrt und dass die Wärmezufuhr den zu verdampfenden Stoff (Festkörper oder Flüssigkeit) und die zu zerstäubende Flüssigkeit gleichzeitig und gemeinsam erhitzt.

12. Vorrichtung zur Zerstäubung einer Flüssigkeit durch einen antreibenden Dampf nach Anspruch 11, **dadurch gekennzeichnet, dass** die Temperaturen der beiden Flüssigphasen (zu verdampfende und zu zerstäubende Flüssigkeit) vor dem Verdampfen bzw. Zerstäuben ungefähr übereinstimmen.

13. Vorrichtung zur Zerstäubung einer Flüssigkeit durch einen antreibenden Dampf nach Anspruch 12, **dadurch gekennzeichnet, dass** die erforderliche zugeführte Wärmeleistung, um die gemeinsame Temperatur der beiden Flüssigkeiten aufrechtzuerhalten, als Steuerparameter für den Druck und die Flussrate des Dampfes dient, der unter Druck durch den Einspritzeinlass (5) eingeleitet wird.

14. Vorrichtung zur Zerstäubung einer Flüssigkeit durch einen antreibenden Dampf nach den Ansprüchen 8 bis 13, **dadurch gekennzeichnet, dass** sich die zu zerstäubende Flüssigkeit zunächst in festem Aggregatzustand befindet und die Wärmeenergie lokal eingeleitet wird, um diesen Feststoff zu schmelzen.

15. Vorrichtung zur Zerstäubung einer Flüssigkeit durch einen antreibenden Dampf nach den Ansprüchen 14, **dadurch gekennzeichnet, dass** man den Feststoff, der in eine zu zerstäubende Flüssigkeit umzuwandeln ist, und den verdampfbaren Stoff (Flüssigkeit oder Feststoff), aus dem man den antreibenden Dampf erzeugt, gemeinsam und durch eine geeignete Wand getrennt aufbewahrt und gemeinsam auf näherungsweise gleiche Temperaturen erwärmt, um die Betriebsbedingungen herzustellen.

16. Vorrichtung zur Zerstäubung einer Flüssigkeit durch einen antreibenden Dampf nach Anspruch 15, **dadurch gekennzeichnet, dass** die erforderliche zugeführte Wärmeleistung, um die gemeinsame Temperatur der beiden Flüssigphasen aufrechtzuerhalten, als Steuerparameter für die Oberflächenspannung und die Viskosität der zu zerstäubenden Flüssigkeit dient.

17. Verfahren zur Zerstäubung einer Flüssigkeit mithilfe eines unter Druck stehenden antreibenden Gases gemäß der Vorrichtung der Ansprüche 1 bis 16.

18. Verfahren zur Zerstäubung einer Flüssigkeit mithilfe eines antreibenden Gases nach Anspruch 17, **dadurch gekennzeichnet, dass** man den erzeugten Sprühnebel oder die Schwebetröpfchen zur Befeuchtung oder Klimatisierung von Innen- und Außenräumen verwendet.

19. Verfahren zur Zerstäubung einer Flüssigkeit mithilfe eines antreibenden Gases nach Anspruch 17, **dadurch gekennzeichnet, dass** die zerstäubte Flüssigkeit Zusatzstoffe enthält, die dazu dienen, die Luft zu akklimatisieren oder zu kühlen, balsamische Stoffe zu zerstäuben, Insekten zu bekämpfen oder durch die Luft beförderte Infektionskrankheiten biologisch zu kontrollieren, oder durch andere Anwendungen gekennzeichnet, für welche die Luft das Hauptmedium bildet, das die Tröpfchen oder ihre Reste nach Verdampfen der Flüssigkeit oder des Lösungsmittels zu ihren Zielbereichen transportiert, beispielsweise zu den Atemwegen eines Lebewesens oder zu seiner Haut oder Augen.

20. Verfahren zur Zerstäubung einer Flüssigkeit mithilfe eines antreibenden Gases nach Anspruch 17, **dadurch gekennzeichnet, dass** man den Sprühnebel oder die Schwebetröpfchen zur Lebensmittelproduktion einsetzt.

21. Verfahren zur Zerstäubung einer Flüssigkeit mithilfe eines antreibenden Gases nach Anspruch 17, **dadurch gekennzeichnet, dass** die Flüssigkeit ein Treibstoff und das Gas verbrennungsfördernd ist, wobei es sich beim Gas des Sprühnebels oder bei den Schwebetröpfchen um eine Mischung für einen Verbrennungsmotor handelt.

22. Verfahren zur Zerstäubung einer Flüssigkeit mithilfe eines antreibenden Gases nach Anspruch 17, **dadurch gekennzeichnet, dass** die Flüssigkeit eine Anstrichfarbe, eine Politur oder ein Hautschutzmittel ist, wobei man den erzeugten Sprühnebel bzw. die Schwebetröpfchen zum Auftragen der genannten Anstrichfarbe, Politur oder des Hautschutzes auf eine außen befindliche Fläche verwendet.

23. Verfahren zur Zerstäubung einer Flüssigkeit mithilfe eines antreibenden Gases nach Anspruch 17, **dadurch gekennzeichnet, dass** die Flüssigkeit eine Probe zur atomspektroskopischen Untersuchung bzw. Bestimmung chemischer Elemente ist.

24. Verfahren zur Zerstäubung einer Flüssigkeit mithilfe eines antreibenden Gases nach Anspruch 17, **dadurch gekennzeichnet, dass** es sich bei der Flüssigkeit um ein Pestizid, Insektizid oder einen sonstigen Stoff handelt, den man im landwirtschaftlichen Pflanzenschutz verwendet.

25. Verfahren zur Zerstäubung einer Flüssigkeit mithilfe eines antreibenden Gases nach Anspruch 17, **dadurch gekennzeichnet, dass** man den Sprühnebel bzw. die Schwebetröpfchen einsetzt, um ein zur maschinellen Bearbeitung von Metallen, Polymeren oder keramischen Materialien verwendetes Werkzeug sowie die bearbeitete Zone zu kühlen.

26. Verfahren zur Zerstäubung einer Flüssigkeit mithilfe eines antreibenden Gases nach Anspruch 17, **dadurch gekennzeichnet, dass** man die Sprühtröpfchen als Mittel verwendet, um in der Umgebungsluft vorhandene Pulver, bestimmte Moleküle oder sonstige Teilchen aller Art volumetrisch aufzunehmen.

## Revendications

1. Dispositif pour pulvériser un liquide au moyen d'une impulsion de gaz ou vapeur déjà pressurisée dans ledit dispositif, les deux fluides étant expulsés vers l'extérieur à la manière d'un aérosol ou de gouttelettes en suspension transporté(s) par ledit gaz ou lesdites vapeurs, le dispositif consistant en une chambre de stockage de liquide (1), contenue dans un réservoir ou une bouteille sous pression (2) sous la pression de la pressurisation commune du gaz et du liquide (p0) ; ledit réservoir (2) étant étanche et incluant une entrée d'injection (5) qui permet d'introduire le gaz ou la vapeur sous pression et un orifice de sortie externe (3) pour le mélange de gaz/liquide ; l'orifice de sortie étant situé dans une zone de mélange (4) où la combinaison des phases gazeuse et liquide a lieu, tout autant que la rupture du débit du liquide et sa sortie vers l'extérieur à la manière d'un aérosol ; l'impulsion de gaz ou vapeur, une fois entrée dans le réservoir (2) à travers l'entrée d'injection (5), sort dans l'environnement à travers l'orifice de sortie (3) ; la surface libre du liquide à l'intérieur du réservoir (2) étant soumise à la pression de l'impulsion de gaz ou de vapeur, de sorte que le liquide soit propulsé vers la zone de mélange à travers un tube d'alimentation (6) dont l'entrée d'aspiration (7) est située près du fond du récipient (2) ; l'autre embout du tube d'alimentation, le dénommé embout du nébuliseur (8), consiste en un trou de sortie (9) dont le bord extérieur (10) fait quasiment face au bord intérieur (11) de l'orifice de sortie (3), formant ainsi entre les deux bords qui se font face une courte différence axiale qui définit une section de passage de forme annulaire pour l'impulsion de gaz ou de vapeur ; ledit orifice de sortie (3) étant percé dans une paroi du réservoir (2) et approximativement situé sur un plan perpendiculaire à l'axe de l'embout du nébuliseur (8) ; le gaz ou la vapeur frappant contre le réservoir pressurisé et traversant radialement ladite section de forme annulaire comprise entre le bord intérieur (11) de l'orifice de sortie (3) et le bord extérieur (10) du trou de sortie (9) avant de sortir à l'extérieur de l'orifice de sortie (3), ledit passage de section de forme annulaire étant la section minimale dans la trajectoire radiale du gaz ou de la vapeur et sa zone étant de l'ordre de la zone de l'orifice de sortie (3) ; la pression (p1) dans le trou de sortie du tube d'alimentation (6) étant comprise entre les valeurs de la pression de la salle extérieure (pa) et la pression de la pressurisation commune du gaz et du liquide (p0), **caractérisé par le fait que** le récipient sous pression (2) inclut une cloison (12) qui le divise en deux compartiments ; tout le liquide étant contenu dans le compartiment inférieur (14), où se trouve un robinet de purge de gaz (16) ouvert vers l'environnement ; l'entrée d'injection (5) du gaz ou de la vapeur sous pression étant située dans le compartiment supérieur (13) ; les deux compartiments étant raccordés l'un à l'autre par le biais d'un orifice de raccordement (15) situé dans la cloison (12) ; ledit orifice de raccordement (15) déterminant une perte de charge dans le gaz ou la vapeur circulant entre les deux compartiments ; le tube d'alimentation (6) traversant ladite cloison (12), de manière que le passage à travers la cloison qui en résulte soit étanche ; trois phases opérationnelles se distinguent : nébulisation, avec robinet fermé (16) : nettoyage ou purge, avec robinet ouvert (16) et retour de flux de gaz dans le tube d'alimentation (6) du trou de sortie (9) iusqu'à l'entrée d'aspiration (7) ; nébulisation contrôlée, avec le robinet dans une position partiellement fermée (16).

2. Dispositif pour la pulvérisation d'un liquide au moyen d'une impulsion de gaz selon la revendication 1, avec pour caractéristique que le compartiment supérieur (13) est divisé en deux réceptacles : un réceptacle de pressurisation (13a) raccordé directement à l'entrée du gaz pressurisé (5) et un réceptacle d'impulsion (13b) qui entoure ladite zone de mélange, les deux réceptacles étant raccordés l'un à l'autre à travers un second orifice de raccordement (15a) qui crée une perte de charge dans le flux de gaz entre les deux réceptacles.

3. Dispositif pour la pulvérisation d'un liquide au moyen d'une impulsion de gaz selon les revendications 1 et 2, avec pour caractéristique que la face extérieure de la paroi du réservoir (2) à proximité du trou de sortie (3) est coupée de manière à créer un cratère à peu près conique dont le bord coïncidera avec le bord intérieur (11) de la section de l'orifice sur la face intérieure de ladite cloison.

4. Dispositif pour la pulvérisation d'un liquide au moyen d'une impulsion de gaz selon les revendications 1 à 3, avec pour caractéristique que les bords de l'orifice et du trou (10 et 11), ou leurs environs proches peuvent présenter quelques finitions superficielles rugueuses, sciées, ondulées d'une taille caractéristique inférieure au diamètre moyen *dj* du jet liquide s'écoulant par l'orifice de sortie après la sortie du liquide dudit trou de sortie (9), et supérieure à l'épaisseur de la couche limite de gaz sur les parois solides.

5. Dispositif pour la pulvérisation d'un liquide au moyen d'une impulsion de gaz selon les revendications 1 à 4, avec pour caractéristique que le réservoir pressurisé (2) comprend deux parties raccordées l'une à l'autre, tout le liquide étant contenu dans l'une des parties, et le réservoir de liquide étant une partie amovible destinée au nettoyage ou au chargement.

6. Dispositif pour la pulvérisation d'un liquide au moyen d'une impulsion de gaz selon la revendication 5, avec pour caractéristique que les deux parties qui composent le réservoir sont jointes par un fil, une pression ou au moyen d'une fermeture, un ajustement ou un élément de fixation externe.

7. Dispositif pour la pulvérisation d'un liquide au moyen d'une impulsion de gaz selon les revendications 1 à 6, avec pour caractéristique que le tube d'alimentation (6) incorpore un contrôle de l'orifice de raccordement.

8. Dispositif pour la pulvérisation d'un liquide au moyen d'une impulsion de gaz selon les revendications 1 à 7, avec pour caractéristique que le gaz pressurisé est une vapeur obtenue à partir d'un liquide ou d'un solide vaporisable.

9. Dispositif pour la pulvérisation d'un liquide au moyen d'une impulsion de vapeur selon la revendication 8, avec pour caractéristique que le liquide vaporisable est choisi parmi les substances suivantes ou une association d'entre elles : eau, alcools, chlorofluorocarbones (CFC), cétones, éthers, esters, paraffine, alcanes, cycloparaffine, naphtènes ou cycloalcanes ou hydrocarbures aromatiques, oléfines, alcènes et autres hydrocarbures insaturés.

10. Dispositif pour la pulvérisation d'un liquide au moyen d'une impulsion de vapeur selon les revendications de 8 à 9, avec pour caractéristique que la vaporisation dudit liquide ou solide vaporisable est faite au moyen d'un approvisionnement de chaleur.

11. Dispositif pour la pulvérisation d'un liquide au moyen d'une impulsion de vapeur selon la revendication 10, avec pour caractéristique que les substances pulvérisables et vaporisables sont stockées dans le dispositif avec une séparation appropriée ; et que l'approvisionnement de chaleur chauffe en même temps le liquide ou le solide à vaporiser et le liquide à pulvériser.

12. Dispositif pour la pulvérisation d'un liquide au moyen d'une impulsion de vapeur, selon la revendication 11, avec pour caractéristique que la température des deux phases liquides, pulvérisables et vaporisables, avant leur vaporisation ou pulvérisation, est environ la même.

13. Dispositif pour la pulvérisation d'un liquide au moyen d'une impulsion de vapeur selon la revendication 12, avec pour caractéristique que la puissance calorifique fournie pour maintenir une température commune dans les deux phases liquides est utilisée comme un paramètre de contrôle de la pression, et que le débit de vapeur est inséré sous pression par l'entrée d'injection (5).

14. Dispositif pour la pulvérisation d'un liquide au moyen d'une impulsion de vapeur selon les revendications 8 à 13, avec pour caractéristique que le liquide à pulvériser est dans une phase solide, et que l'énergie calorifique est fournie localement pour produire la fusion de la phase solide.

15. Dispositif pour la pulvérisation d'un liquide au moyen d'une impulsion de vapeur selon la revendication 14, avec pour caractéristique que la phase solide censée devenir pulvérisable et le liquide ou solide vaporisable censé devenir vapeur à impulsion sont stockés ensemble moyennant une cloison adaptée, et sont chauffés simultanément à une température à peu près commune aux deux pour garantir l'établissement de conditions opérationnelles.

16. Dispositif pour la pulvérisation d'un liquide au moyen d'une impulsion de vapeur selon la revendication 15, avec pour caractéristique que la puissance calorifique fournie pour conserver une température commune dans les deux phases liquides est utilisée comme un paramètre de contrôle pour la tension superficielle et la viscosité du liquide à pulvériser.

17. Procédure pour la pulvérisation d'un liquide au moyen d'une impulsion de gaz selon les revendications du dispositif 1 à 16.

18. Procédure pour la pulvérisation d'un liquide au moyen d'une impulsion de gaz selon la revendication 17, avec pour caractéristique que l'aérosol ou les gouttelettes en suspension généré(es) est(sont) utilisé(es) pour l'humidification ou l'acclimatation des espaces intérieurs comme extérieurs.

19. Procédure pour la pulvérisation d'un liquide au moyen d'une impulsion de gaz selon la revendication 17, avec pour caractéristique que le liquide à pulvériser contient des additifs visant à l'acclimatation de l'air, l'assainissement de l'air, la dispersion de substances balsamiques, la désinsectisation, le contrôle biologique des maladies infectieuses transportées par l'air et d'autres applications où l'air atmosphérique constitue le véhicule de transport général des gouttelettes ou de leurs restes, après évaporation du liquide ou solvant vers les zones d'objectif finales, lesquelles peuvent être le système respiratoire de tout être vivant, sa peau ou ses yeux.

20. Procédure pour la pulvérisation d'un liquide au moyen d'une impulsion de gaz selon la revendication 17, avec pour caractéristique que l'aérosol ou les gouttelettes en suspension généré(es) est(sont) utilisé(es) dans la production de nourriture.

21. Procédure pour la pulvérisation d'un liquide au moyen d'une impulsion de gaz selon la revendication 17, avec pour caractéristique que le liquide est un combustible et le gaz un comburant, et le gaz à aérosol ou les gouttelettes en suspension est(sont) un mélange dans un moteur à combustion interne.

22. Procédure pour la pulvérisation d'un liquide au moyen d'une impulsion de gaz selon la revendication 17, avec pour caractéristique que le liquide est une peinture, une cire ou une protection de la peau, et l'aérosol ou les gouttelettes en suspension est(sont) utilisé(es) dans l'application de ladite peinture, cire ou protection de la peau sur une surface située à l'extérieur.

23. Procédure pour la pulvérisation d'un liquide au moyen d'une impulsion de gaz selon la revendication 17, avec pour caractéristique que le liquide est un échantillon pour une analyse élémentaire réalisée au moyen de techniques de spectrométrie atomique.

24. Procédure pour la pulvérisation d'un liquide au moyen d'une impulsion de gaz selon la revendication 17, avec pour caractéristique que le liquide est un pesticide, insecticide ou tout autre substance utilisée dans le secteur agricole de traitement des cultures.

25. Procédure pour la pulvérisation d'un liquide au moyen d'une impulsion de gaz selon la revendication 17, avec pour caractéristique que l'aérosol ou les gouttelettes en suspension est(sont) utilisé(es) pour refroidir la zone des machines et l'outil lors de toute action des machines sur les matériaux métalliques, polymères ou céramiques.

26. Procédure pour la pulvérisation d'un liquide au moyen d'une impulsion de gaz selon la revendication 17, avec pour caractéristique que l'aérosol est utilisé comme un moyen de collecte volumétrique de poussière, particules de toute nature, ou molécules en suspension dans l'environnement où l'aérosol est répandu.
